(19) **Europäisches Patentamt**

**European Patent Office**

**Office européen des brevets**

(11) Veröffentlichungsnummer: **0 212 475**
**B1**

(12) # EUROPÄISCHE PATENTSCHRIFT

(45) Veröffentlichungstag der Patentschrift:
25.01.89

(21) Anmeldenummer: 86110937.9

(22) Anmeldetag: 07.08.86

(51) Int. Cl.⁴: **A61B 17/44**

(54) **Vakuum-Extraktor zur Geburtshilfe.**

(30) Priorität: **07.08.85 DE 3528397**
**01.10.85 DE 3535055**

(43) Veröffentlichungstag der Anmeldung:
**04.03.87 Patentblatt 87/10**

(45) Bekanntmachung des Hinweises auf die Patenterteilung:
**25.01.89 Patentblatt 89/4**

(84) Benannte Vertragsstaaten:
**AT BE CH DE FR GB IT LI LU NL SE**

(56) Entgegenhaltungen:
**DE-A- 2 720 973**
**FR-A- 1 208 805**
**GB-A- 839 327**
**GB-A- 1 331 587**
**US-A- 2 702 038**
**US-A- 3 848 606**

(73) Patentinhaber: **Hepp, Herrmann, Prof. Dr. med.,,**
**Marchioninistrasse 15, D-8000 München 70(DE)**
Patentinhaber: **King, Siegfried, Dr. med. Ing. grad.,,**
**Lerchenweg 3, D-8027 Neuried(DE)**

(72) Erfinder: **King, Siegfried, Dr.med.Ing.grad.,**
**Lerchenweg 3, D-8027 Neuried(DE)**

(74) Vertreter: **von Puttkamer, Nikolaus, Dipl.-Ing.,**
**Pienzenauerstrasse 2, D-8000 München 80(DE)**

## Beschreibung

Die vorliegende Erfindung betrifft einen Vakuum-Extraktor zur Geburtshilfe nach dem Oberbegriff des Patentanspruches 1.

Die Vakuum-Extraktion ist zur operativen, vaginalen Geburtshilfe seit Jahren anerkannt. Dabei zur Anwendung gelangende, bekannte Vorrichtungen, wie der beispielsweise aus der US-PS 2 702 038 bekannte Vakuum-Extraktor, bestehen aus einem evakuierbaren Behälter, dessen Öffnung nach dem Einführen in die Scheide einer Gebärenden am Kopf des Kindes angesetzt wird. Der Kopf saugt sich bei der Erzeugung eines Unterdruckes im Behälter unter Ausbildung einer Gewebsgeschwulst am Behälter fest, so daß dann über einen Haltegriff und gezielte Zugbewegungen der Geburtsvorgang unterstützt und auch beschleunigt werden kann.

Die Vakuum-Extraktion ist jedoch, wie andere instrumentelle Entbindungshilfen auch nicht risikofrei. Bei zu starkem Zug und insbesondere beim Auftreten von Zugkräften, die tangential zum Behälter verlaufen, kann der am Kopf eines Kindes angesaugte Behälter unbeabsichtigt und abrupt von der Kopfschwarte abreißen, was zu erheblichen intrakraniellen Druckschwankungen führen kann. Es wurden daher wiederholt die Handhabung und die Konstruktion des Extraktors betreffende Verbesserungen vorgeschlagen.

So ist beispielsweise aus der US-PS 2 702 038 (Fig. 6) ein Vakuum-Extraktor bekannt, bei dem ein starrer Handgriff über ein Kugelgelenk mit dem Behälter verbunden ist, um ein besseres Placieren und Dirigieren des Vakuum-Extraktors zu ermöglichen. Ein derartiges Kugelgelenk läßt jedoch unter Zug keine reibungsfreie Verschwenkung des starren Haltegriffes und keine freie Rotation des Kopfes zu, da es zu einem Kraftschluß im Gelenk kommt. Dies führt wiederum dazu, daß das Herausleiten des kindlichen Kopfes aus dem Geburtskanal wesentlich erschwert wird.

Es ist auch ein Vakuum-Extraktor bekannt, bei dem die Kraft gemessen, mit der die Kopfschwarte beim Evakuieren des Behälters gegen eine im Inneren des Behälters angeordnete Andruckplatte drückt. Durch die Messung dieser Andruckkraft kann jedoch ein möglicher Gefahrenzustand, bei dem das Abreißen des Behälters erfolgt, nur unzulänglich vorhergesagt werden, weil die Andruckkraft der Kopfschwarte in Abhängigkeit von aus unterschiedlichen Richtungen am Behälter angreifenden Zugkräften in einer völlig unvorhersehbaren Weise verändert wird.

Die aus der DE-PS 3 138 589 bekannte Anordnung, bei der schädliche Druckschwankungen beim Abreißen des Behälters dadurch vermindert werden sollen, daß eine im Inneren des Behälters angeordnete Gewebehaltevorrichtung ein Zurückschwingen der Kopfschwarte verzögert, ist nachteilig, weil bei einem derartig beschaffenen Behälter nur relativ kleine Zugkräfte übertragen werden können.

Allgemein kann gesagt werden, daß bei allen bekannten Vakuum-Extraktoren ein Problem dahingehend besteht, daß das im Behälter aufgebaute Vakuum bei der Ausübung von Zugkräften, insbesondere bei nicht koaxial gerichteten Zugkräften, abrupt zusammenbricht, wenn am Randbereich der Behälteröffnung eine Verbindung zwischen dem evakuierten Bereich und der Umgebung hergestellt wird. Neben der errechenbaren maximalanwendbaren Zugkraft, die vom Durchmesser des evakuierten Behälters abhängig ist, spielen Parameter, wie insbesondere die Zugrichtung, das Kippmoment und die Gewebseigenschaften der Kopfgeschwulst eine entscheidende Rolle, weswegen letztlich Zug- und Druckmessungen allein nicht verläßlich genug einen drohenden Abriß anzeigen können, sofern bei der Auswertung der Meßergebnisse nicht eine Sicherheitszone berücksichtigt wird. Die Berücksichtigung einer solchen Sicherheitszone würde aber zu einer deutlichen Begrenzung der einsetzbaren Zugkräfte führen.

Die Aufgabe der vorliegenden Erfindung besteht daher darin, einen wie eingangs genannten Vakuum-Extraktor zur Geburtshilfe dahingehend zu verbessern, daß ein plötzlicher und vollständiger Zusammenbruch des im Extraktor aufgebauten Vakuums vermieden werden kann, ohne daß dabei auf die optimal einsetzbaren Zugkräfte verzichtet werden muß.

Diese Aufgabe wird durch einen Vakuum-Extraktor der eingangs genannten Art gelöst, der durch die in dem kennzeichnenden Teil des Patentanspruches 1 angegebenen Merkmale gekennzeichnet ist.

Der wesentliche Vorteil der vorliegenden Erfindung besteht darin, daß ein plötzlicher und abrupter Zusammenbruch des im Extraktor aufgebauten Vakuums vermieden werden kann, weil im Extraktor wenigstens zwei getrennt voneinander evakuierbare Behälterkammern vorgesehen sind. Vorzugsweise sind im Extraktor zwei konzentrisch zueinander angeordnete Behälterkammern vorgesehen, die über zwei Vakuum-Pumpen evakuiert werden. Der zu unterstützende Geburtsvorgang kann daher vorteilhafterweise nicht durch ein unerwartetes, abruptes und vollständiges Abreißen des Extraktors vom Kopf des Kindes kompliziert werden, weil in dem Fall, in dem auf dem äußeren Behälter des Extraktors eine zu starke oder fehlerhafte Zugkraft ausgeübt wird, zunächst nur die schmale, niedrig dimensionierte Gewebsgeschwulst in der äußeren Kammer die vollständige Abdichtung des Vakuums der äußeren Kammer nicht mehr aufrechterhalten kann, wobei der Zusammenbruch des Vakuums in der äußeren Kammer lange vor dem Zustand erfolgt, bei dem die angewandten Zugkräfte übertragende innere Behälterkammer sich von der erzeugten eigentlichen Gewebsgeschwulst zu lösen beginnt. Der Zusammenbruch des Vakuums in der äußeren Kammer, der bei einer bevorzugten Ausführungsform der Erfindung über einen Signalgeber angezeigt wird, zeigt daher zuverlässig den kritischen Punkt vor dem Abreißen der inneren Behälterkammer von der Kopfschwarte an. Während dieser kritische Punkt angezeigt wird, hält die innere Behälterkammer vorteilhafterweise die Verbindung zum Kopf des Kindes weiter aufrecht, bis unter erneutem Andruck des vorzugsweise starren Systems an den kindlichen Kopf auch das Vakuum in der äußeren Behälterkammer wieder aufgebaut ist und eine erneute vehensynchrone Extraktion wieder ermöglicht wird.

Ein wesentlicher Vorteil einer Weiterbildung der Erfindung besteht darin, daß infolge der Vorsehung einer zentralen, niedrig gehaltenen Deckplatte der Behälterkammern das artifizielle caput succedaneum im mittleren Bereich der Behälterkammern relativ flach gehalten und daher insgesamt minimiert werden kann, wohingehend das caput succedaneum im peripheren Bereich in seitlichen Ausbuchtungen der Behälterkammern verstärkt aufgebaut und daher optimiert werden kann. Dadurch kann der für die Extraktion notwendige Kraftschluß mit dem kindlichen Kopf vorteilhafterweise verstärkt werden. Auf diese Weise wird, ohne daß auf die notwendige Gewebsschulter der angesaugten Kopfschwarte verzichtet werden muß, das Kind weniger traumatisiert. Zudem wird die Extraktion vorteilhafterweise früher möglich, weil die notwendige Gewebsgeschwulst insgesamt ein kleineres Volumen erfordert.

Der Vorteil einer Weiterbildung der Erfindung, bei der der Haltegriff von den Behältern in einer speziellen Weise mechanisch entkoppelt ist, besteht darin, daß der kindliche Kopf während des Geburtsvorganges selbst unter Zug seine notwendige freie Beweglichkeit im Geburtskanal beibehalten kann, wobei aber gleichzeitig, sofern dies zur schonenden Geburtshilfe notwendig ist, über den vorzugsweise starren Haltegriff eine aktive Rotation des kindlichen Kopfes möglich ist.

Ein weiterer wesentlicher Vorteil einer bevorzugten Ausführungsform des erfindungsgemäßen Vakuum-Extraktors, bei dem der Halte- bzw. Zuggriff über ein spezielles Kugellagergelenk nahezu reibungsfrei mit dem Behälter verbunden ist, besteht darin, daß insbesondere dann, wenn ein nicht koaxialer Zug über den Haltegriff ausgeübt wird, der äußere Behälterrand keine Kippbewegungen ausführt, weil bei schräg bzw. tangential am Behälter angreifenden Zugkräften die Kugeln des Kugellagergelenkes, die die Zugkräfte vom Haltegriff auf die Behälter übertragen, verschwenkt werden, wobei sich die an den Behältern angreifenden Kraftvektoren so verändern, daß der normalerweise infolge der aufgebrachten Zugkomponente vom Abreißen bedrohte Rand der Behälter weniger belastet wird. Dadurch kann vorteilhafterweise die Gefahr des Abreißens erheblich verringert werden. Aufgrund der mechanischen Entkopplung wird vorteilhafterweise gleichzeitig neben der über den Haltegriff möglichen Zug- bzw. Druckbewegung während der Extraktion eine nahezu reibungsfreie Drehung des kindlichen Kopfes sichergestellt, wobei sich der Kopf vorteilhafterweise, wie bei einer Spontangeburt, den Gegebenheiten des Geburtskanales nach den Gesetzen des geringsten Widerstandes optimal anpassen kann. Die Drehbewegung kann gemäß einer Weiterbildung der Erfindung durch die Vorsehung entsprechender Anschlagpunkte im Kugellagergelenk auf einen vorgegebenen Bereich begrenzt sein, so daß bedarfsweise, falls dies während des Geburtsvorganges gewünscht wird, eine aktive Drehung des Köpfchens durch Ausführung einer Drehbewegung am Haltegriff bewirkt werden kann.

Bei einer weiteren Ausgestaltung der Erfindung ist der Haltegriff von den Behältern abnehmbar. Dies hat den Vorteil, daß am Haltegriff verschieden große Behälter angebracht werden können. Zudem wird dadurch auch eine vereinfachte Sterilisation der Behälter des Vakuum-Extraktors ermöglicht.

Bei einer weiteren vorteilhaften Ausgestaltung der Erfindung sind die Handgriffteile des Haltegriffes über ein Gelenk verschwenkbar am Haltegriff befestigt, so daß sie beim Einführen des Vakuum-Extraktors in Richtung auf die Zugstange des Haltegriffes eingeklappt werden können, um Behinderungen bei der Handhabung möglichst zu vermeiden.

Bei einer weiteren vorteilhaften Ausgestaltung der Erfindung können während der Extraktion die kindlichen Herzaktionen durch eine mittig im entsprechenden Behälter vorgesehene Sonde erfaßt werden. Beim Stand der Technik ist es zur Ermittlung von Herzaktionen während der Geburt üblich, eine entsprechende Vorrichtung zur Ableitung der Herzaktionen am kindlichen Kopf anzubringen. Derartige Vorrichtungen müssen dann vor Anlegen des Extraktors wieder entfernt oder, sofern dies möglich ist, seitlich vom Extraktor erneut am Kopf des Kindes angebracht werden. Im ersten Fall führt dies dazu, daß nach dem Anbringen des Extraktors überhaupt keine für die Überwachung des Geburtsvorganges aufzuzeichnenden, intern abgeleitete Herzaktionen mehr ermittelt werden können. Im zweiten Fall sind für die Anbringung der Vorrichtung seitlich vom Kopf des Behälters des Extraktors zeitraubende Schritte erforderlich.

Außerdem zeigt der zustande gekommene Kontakt mit der Sonde vorteilhafterweise an, daß sich im entsprechenden Behälter eine die Einleitung der Extraktion ermöglichende hinreichend große Gewebsgeschwulst ausgebildet hat.

Im folgenden werden die Erfindung und deren Ausgestaltungen im Zusammenhang mit den Figuren näher erläutert. Es zeigt:

Fig. 1 eine schematische Darstellung einer einfachen Ausführungsform des erfindungsgemäßen Vakuum-Extraktors;

Fig. 2 eine weitere Ausführungsform des erfindungsgemäßen Vakuum-Extraktors;

Fig. 3 und 4 eine weitere Ausführungsform des erfindungsgemäßen Vakuum-Extraktors, bei dem eine mechanische Entkopplung zwischen dem Handgriff und den Behältern vorgesehen ist; und

Fig. 5 zur Verdeutlichung der Funktion der mechanischen Entkopplung einem am Kopf des Kindes angesetzten Vakuum-Extraktor gemäß Fig. 3, wobei sich der Kopf des Kindes in dem gebogenen Geburtskanal befindet.

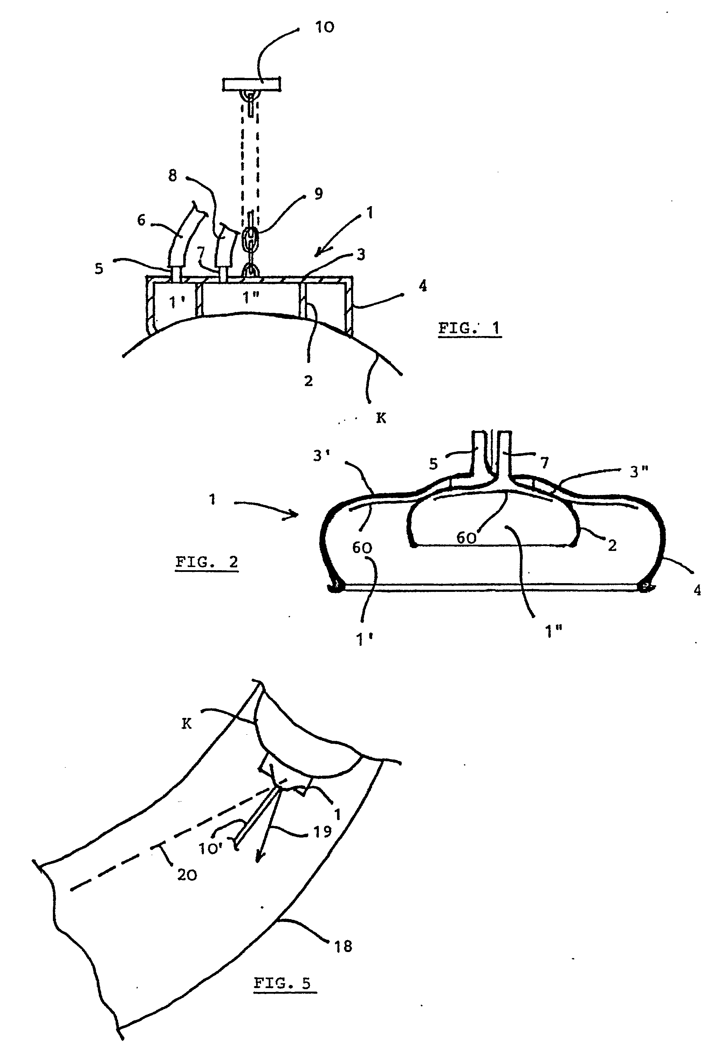

In der schematischen Darstellung der Figur 1 ist der evakuierbare Behälter des vorliegenden Vakuum-Extraktors mit 1 bezeichnet. Im wesentlichen besteht dieser Behälter aus einer oberen Wand 3, von der aus sich eine vorzugsweise ringförmig ausgestaltete Seitenwand 4 nach unten erstreckt. Im Behälter 1 sind mehrere, vorzugsweise zwei Behälterkammern 1', 1" vorgesehen, die vorzugsweise konzentrisch zueinander angeordnet sind. Dabei sind die Behälterkammern 1', 1" durch eine weitere ringförmige Wand 2 voneinander getrennt, die sich von der oberen Wand 3 aus nach unten erstreckt und konzentrisch zu der Seitenwand 4 angeordnet ist. Die äußere ringförmige Behälterkammer 1' steht über einen dicht durch die obere Wand 3 hindurchgeführten Saugstutzen 5 mit einem Absaugschlauch 6 in Verbindung. In der entsprechenden Weise steht die innere Behälterkammer 1" über einen dicht durch die obere Wand 3 hindurchgeführten Saugstutzen 7 mit einem Absaugschlauch 8 in Verbindung. Über die Absaugschläuche 6 und 8, die zu nicht dargestellten, externen Vakuumpumpen führen, die in einer Vakuumeinrichtung zusammengefaßt sein können, sind die Behälterkammern 1', 1" getrennt evakuierbar. Dabei ist es vorzugsweise möglich, in der inneren Behälterkammer 1", die sich nach dem Ansetzen des Behälters 1 am Kopf eines Kindes im Bereich der Fontanelle befindet, ein kleineres Vakuum als in der äußeren Behälterkammer, die nicht im Bereich der Fontanelle liegt, zu erzeugen. Beispielsweise werden in der inneren Behälterkammer 1" ein Vakuum von 0,8 kp/cm$^2$ und in der äußeren Behälterkammer 1' ein Vakuum von z.B. 0,99 kp/cm$^2$ erzeugt. Vorzugsweise verlaufen die Absaugschläuche 6, 8 entlang der Kette 9. An der Stelle der Kette 9 kann auch eine andere Zugvorrichtung vorgesehen sein. Die Längen der ringförmigen Seitenwand 4 und der ringförmigen Wand 2 sind zweckmäßigerweise so bemessen, daß die freien Enden der Wände 4 und 2 beim Anlegen des Vakuum-Extraktors an dem Kopf K des Kindes an diesem anliegen. Dies bedeutet, daß die Länge der inneren Wand 2 entsprechend der Wölbung des Kopfes des Kindes kürzer ist als die Länge der äußeren Wand 4. Bei der Anwendung des erfindungsgemäßen Vakuum-Extraktors entstehen lediglich vergleichsweise kleine Gewebsgeschwülste, weil sich die Kopfhaut des Kopfes des Kindes zusätzlich an dem freien Ende der inneren ringförmigen Wand 2 abstützt.

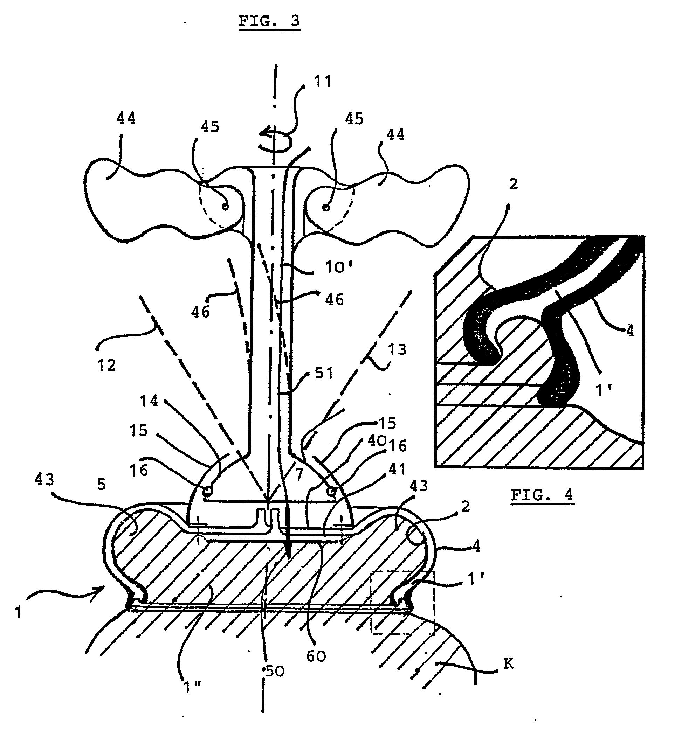

Aus der Figur 2 geht ein Behälter 1 hervor, bei dem die Bewegungsfreiheit beim Arbeiten mit dem Vakuum-Extraktor voll erhalten bleibt, weil die beiden Absaugstutzen 5, 7 annähernd mittig bzw. symmetrisch zur Achse des Behälters 1 angeordnet sind. Einzelheiten der Figur 2, die bereits im Zusammenhang mit der Figur 1 erläutert wurden, tragen die entsprechenden Bezugszeichen. Die symmetrische Anordnung der Absaugstutzen 5, 7 in Bezug auf die Längsachse des Behälters 1 ist dadurch möglich, daß an der Stelle der oberen Wand 3 des Behälters der Figur 1 eine Doppelwand 3', 3" vorgesehen wird, wobei die einzelnen Wände 3', 3" dieser Doppelwand übereinander ausgebildet werden und wobei zwischen der oberen Wand 3' und der darunter angeordneten Wand 3" ein Kanal besteht, der den Ansaugstutzen 5 mit der äußeren Behälterkammer 1' verbindet. Der dicht durch die obere Wand 3' hindurchgeführte Absaugstutzen 7 führt zur inneren Behälterkammer 1". Beispielsweise kann mit diesem Behälter 1 ebenfalls die aus der Figur 1 ersichtliche Kette 9, die eine Verbindung zum Handgriff 10 herstellt, verbunden sein.

Aus der Figur 3 geht eine Weiterbildung der Erfindung hervor, bei der ein Zug- bzw. Haltegriff 10' vom Behälter 1 mechanisch derart entkoppelt ist, daß der Haltegriff 10' in Bezug auf den Behälter 1 eine Kreiselbewegung ausführen kann. Genauer gesagt ist der Haltegriff 10' so ausgebildet, daß er sich einerseits relativ zum Behälter 1 um seine Längsachse drehen kann, wie dies in der Figur 3 durch den Pfeil 11 angedeutet ist, und daß er andererseits in einem vorgegebenen Winkelbereich nach allen Richtungen in Bezug auf die Längsachse des Behälters 1 weitgehend reibungsfrei verschwenkbar ist, wie dies durch die Linien 12, 13 dargestellt ist. Die mechanische Entkopplung wird dadurch erreicht, daß das dem Behälter 1 zugewandte Ende des Haltegriffes 10' ein erweitertes Ende 14 aufweist, das eine sphärische bzw. kugelförmige Außenfläche besitzt. Der Behälter 1 weist eine nach oben führende, ebenfalls kugelförmig ausgestaltete Wand 15 auf, die die kugelförmige Außenfläche des erweiterten Endes 14 des Haltegriffes 10' umgibt. Am erweiterten Ende 14 des Haltegriffes 10' sind schematisch dargestellte Kugellager 16 vorgesehen, durch die sichergestellt wird, daß die kugelförmigen Außenflächen des erweiterten Endes 14 an der kugelförmigen Oberfläche 15 abrollen bzw. gleiten kann. Genauer gesagt wird durch die Kugellager 16 erreicht, daß das erweiterte Ende 14 und der damit verbundene Haltegriff 10' in Bezug auf den Behälter 1 um die Längsachse des Behälters 1 verdreht werden und gleichzeitig auch gegenüber der Längsachse verschwenkt werden kann, wie dies oben bereits erläutert wurde. Beispielsweise kann der Haltegriff 10' in Bezug auf die Längsachse des Behälters 1 um einen Winkel von 30° oder mehr nach allen Richtungen verschwenkt werden. Um Verletzungen bei der Ausführung von Kreiselbewegungen zu vermindern, die durch Einzwicken von mütterlichen Gewebeteilen entstehen können, kann eine vorzugsweise gummiartige Muffe (nicht dargestellt) über dem Bereich angeordnet werden, in dem sich der obere Endbereich der Wand 15 und das erweiterte Ende 14 überlappen. Diese Muffe behindert die Kreiselbewegung nicht.

Bei der dargestellten Ausführungsform ist der Haltegriff 10' vorzugsweise hohl ausgebildet, so daß die mit den Abzugsstutzen 5, 7 verbundenen Schläuche (nicht dargestellt) durch den Haltegriff 10' hindurchgeführt werden können.

Aus der Darstellung der Figur 3 ist auch ersichtlich, daß gemäß einer Weiterbildung der Erfindung der Abstand zwischen den beiden Behälterkammern 2, 4 relativ klein gewählt werden kann, so daß die innere Behälterkammer praktisch die gesamten Haltefunktionen erfüllt und daß die in die äußere Behälterkammer 1' eingesaugte Kopfschwarte (siehe vergrößerte Darstellung der Figur 4) dazu verwendet wird, in der nachfolgend näher erläuterten Weise als Indikator für das drohende Abreißen der inneren Behälterkammer 1" von der Kopfschwarte des kindlichen Kopfes zu dienen. Nach dem Aufsetzen des zuvor erläuterten Behälters des Vakuum-Extraktors der Figur 3 auf die Kopfschwarte K des kindlichen Kopfes werden die beiden Behälterkammern 1' und 1" evakuiert. Dabei wird, etwa in der aus der Figur 4 ersichtlichen Weise, in der die Kopfschwarte schräg schraffiert dargestellt ist, ein Teil der Kopfschwarte in die äußere Behälterkammer 1' eingesaugt. Wenn nun bei der Extraktion Zugkräfte auf den Behälter ausgeübt werden, tritt vor dem Abreißen des Vakuums in der inneren Behälterkammer 1" immer zuverlässig der Fall ein, daß die vor der Ausübung des kritischen Zuges eine vollständige Abdichtung der äußeren Behälterkammer 1' bewirkende kleine Gewebeschwulst in der äußeren Behälterkammer so verformt wird, daß eine Verbindung zwischen dem Vakuum der äußeren Behälterkammer 1' und der Umgebung hergestellt wird. In diesem Fall, in dem die innere Behälterkammer aber noch sicher und fest am Kopf des Kindes angesaugt ist, bricht das Vakuum in der äußeren Behälterkammer 1' zusammen. Durch einen geeigneten Signalgeber wird dieser Zustand dem Geburtshelfer angezeigt, so daß er lange bevor die innere Behälterkammer 1" sich von der erzeugten eigentlichen Kopfgeschwulst zu lösen beginnt, Maßnahmen ergreifen kann, die wieder zum Aufbau des Vakuums in der äußeren Behälterkammer 1' führen. Dies bedeutet, daß durch den Abbau des Vakuums in der äußeren, vergleichsweise kleinvolumigen Kammer zuverlässig angezeigt wird, daß eine Situation eingetreten ist, in der bei einer weiteren Vergrößerung der über die innere Behälterkammer auf die Kopfschwarte K ausgeübten Zugkraft ein Abreißen des Vakuum-Extraktors bevorsteht. Durch geeignete Dimensionierung der Form des Randbereiches der Öffnung der äußeren Behälterkammer 1' und des Abstandes der die äußere Behälterkammer 1' bildenden Wände 2, 4 voneinander kann auch in Abhängigkeit von dem in der äußeren Behälterkammer 1' angewendeten Vakuum eine rechtzeitige Signalgabe vor dem Abreißen des Extraktors rechtzeitig und sicher bewirkt werden.

Dabei ist es von besonderer Bedeutung, daß bis zum Zeitpunkt des Abreißens des Vakuums in der äußeren Behälterkammer 1' aufgrund der zuvor angesprochenen Dimensionierung über die innere Behälterkammer 1" noch der erforderliche maximale Zug ausgeübt werden kann.

Im folgenden wird nun anhand der Figur 5, die einen im Geburtskanal 18 befindlichen Kopf K eines Kindes zeigt, die Funktionsweise des mechanisch entkoppelten Vakuum-Extraktors der Figur 3 näher erläutert. Zunächst wird der Extraktor derart in den Geburtskanal 18 eingeführt, daß die freien Enden der Wände 2 und 4 der Behälterkammer 1' und 1" am Kopf K des Kindes anliegen. Über die durch den Haltegriff 10' und den Endbereich 14 verlaufenden Abzugschläuche werden nun die Behälterkammern 1' und 1" getrennt evakuiert. Dabei saugt sich der gesamte Behälter 1 am Kopf des Kindes fest. Über den Haltegriff 10' und das erweiterte Ende 14 können nun auf den am Kopf K des Kindes befestigten Behälter 1 Zug/Druck-Kräfte ausgeübt werden, um den Kopf des Kindes durch den Geburtskanal 18 zu ziehen bzw. zu führen. Dabei ist es von wesentlicher Bedeutung, daß durch die mechanische Entkopplung zwischen dem Behälter 1 und dem Haltegriff 10' des Vakuum-Extraktors durch geeignetes Drücken am Haltegriff 10' erreicht werden kann, daß auf den Behälter 1 stets ein Kraftvektor 19 ausgeübt wird, der in etwa senkrecht zum Kopf des Kindes verläuft. Dadurch wird erreicht, daß ein Kippen des Behälters 1 in Bezug auf die Oberfläche des Kopfes K des Kindes weitgehend vermindert wird und daß zumindest das in der Kammer 1" aufgebaute Vakuum nicht abbricht. In der Figur 5 ist durch die gestrichelte Linie 20 dargestellt, in welcher Weise bei dem bekannten Vakuum-Extraktor der Vektor der auf den Behälter 1 verlaufenden Zugkraft verläuft. Es ist ohne weiteres ersichtlich, daß die Zugbeanspruchungen, die entlang der Linie 20 verlaufen, dazu führen können, daß der Behälter 1 in der Figur 5 an seiner rechten Seite vom Kopf K abgehoben wird, da die Zugbeanspruchung vorwiegend nicht entlang der Achse des Behälters 1 verläuft. Mit der Hilfe des vorliegenden Extraktors kann durch geeignete Druckausübung über den starren Haltegriff 10' erreicht werden, daß der Winkel zwischen den Längsachsen des Haltegriffes 10' und des Behälters 1 des Extraktors in Abhängigkeit von der Biegung des Geburtskanales 18 immer so beschaffen ist, daß die auf den Behälter 1 einwirkenden Zugkräfte, die durch Ziehen und Drücken am Haltegriff 10' bewirkt werden, etwa senkrecht zur Kopfoberfläche des Kindes verlaufen. Gleichzeitig kann der Kopf die natürlichen und notwendigen Rotationsbewegungen ausführen, weil sich der Behälter 1 infolge der mechanischen Entkopplung in Bezug auf den Haltegriff 10' frei drehen kann.

Vorzugsweise ist an einer geeigneten Stelle ein Fühler (nicht dargestellt) vorgesehen, der zweckmäßigerweise durch Messen der Luftliterleistung der Vakuumpumpe für den äußeren Behälter 1' beim Abreißen des Vakuums in der äußeren Behälterkammer 1' ein Signal für eine Steuereinrichtung (nicht dargestellt) erzeugt, die ihrerseits einen zweckmäßerweise akkustischen Signalgeber erregt, der dem Arzt anzeigt, daß Maßnahmen zu ergreifen sind, die zum Wiederaufbau des Vakuums in der äußeren Behälterkammer 1' führen.

Im folgenden wird im Zusammenhang mit der Figur 3 eine weitere Ausgestaltung der Erfindung erläutert. Für die suffiziente Haftung des Extraktors ist die Ausbildung eines caput succedaneums erforderlich. Die Extraktion sollte daher nicht vor einer ausreichenden "Kopfgeschwulstbildung", die den inneren Behälter des Extraktors im wesentlichen ausfüllt, begonnen werden. Eine ausreichende Ausbildung des caput succedaneums kann bei der Ausführungsform der Figur 3 dadurch erfaßt werden, daß eine Dornanordnung vorgesehen wird, die wenigstens einen Stift 50, der vorzugsweise eine Spitze aufweist und elektrisch leitend ist, umfaßt, der vorzugsweise im mittleren Bereich der inneren Behälterkammer 1" derart angeordnet ist, daß er sich in Richtung auf das offene Ende der Behälterkammer 1" eine vorgegebene Entfernung erstreckt. Der Stift 50 ist in der oberen Behälterwand 3" der inneren Behälterkammer 1" bzw. in der Platte 60 befestigt. Bei der Ausbildung des caput succedaneums wird der Stift 50 einige Millimeter in den angesogenen kindlichen Skalp eingedrückt. Sobald dies geschehen ist, dürften die Voraussetzungen für eine wehensynchrone Extraktion gegeben sein. Der Stift 50 ist über eine schematisch dargestellte elektrische Leitung 51, die durch den Haltegriff 10' verläuft, mit einer externen Meßeinrichtung verbindbar. Sobald sich der Stift 50 in den kindlichen Skalp eingräbt, sind durch die Meßeinrichtung Herzaktionen während der gesamten Extraktionsoperation erfaßbar.

Der voranstehend beschriebene Stift 50 kann auch im Zusammenhang mit herkömmlichen Extraktionen, die lediglich eine Kammer aufweisen, angewendet werden.

Bei den in den Figuren mit dem Bezugszeichen 60 bezeichneten Einrichtungen handelt es sich um an sich bekannte Stützbleche, an denen sich bei der Ausbildung des Vakuums im Extraktor der Kopf des Kindes abstützt. Dadurch wird dafür Sorge getragen, daß der Saugstutzen 7 nicht verschlossen wird.

Bei einer weiteren Ausgestaltung der Erfindung ist der mittlere Bereich 40, 41 der beiden Behälterkammern nach innen abgesenkt, so daß die Gewebsgeschwulst schneller in einem kleinen Volumen aufgebaut wird, weshalb die Extraktion früher eingeleitet werden kann. Zudem breitet sich die Gewebsgeschwulst bei ihrem Aufbau in die ausgehend von der niedrig gehaltenen Platte 40, 41 nach außen führende ringförmige Ausbeulung 43 der Behälterkammern aus, so daß über die über den Öffnungsumfang der inneren Behälterkammer 1" in dem seitlich nach außen hinausragenden Bereich erzeugte Gewebsgeschwulst eine optimale Verbindung zwischen der Gewebsgeschwulst insgesamt und dem Vakuum-Extraktor hergestellt wird.

In der ebenfalls aus der Figur 6 ersichtlichen Weise können die an der Zugstange des Haltegriffes 10' des Vakuum-Extraktors befestigten Handgriffteile 44 über Gelenke 45 mit der Zugstange verbunden sein, so daß sie beim Einführen des Vakuum-Extraktors in die Scheide in Richtung auf die Haltestange geklappt werden können, um nicht störend in Erscheinung zu treten. Nach dem Einführen werden die Handgriffteile 44 in die in der Figur 3 dargestellte Position zurückgeklappt, in der über sie die aufgebrachten Zugkräfte auf die Zugstange übertragen werden können.

Es ist denkbar, die Zugstange des Haltegriffes 10' nicht aus einem völlig starren Rohr, sondern aus einem halbstarren Rohr zu bilden, so daß sie beim Extraktionsvorgang beispielsweise in der in der Figur 3 durch die Linien 46 dargestellte Position verbogen werden kann. Dadurch wird erreicht, daß der Haltegriff 10' in einem noch größeren Winkel, als der beispielsweise dargestellte Winkel, der über die Kugelgelenkanordnung ermöglicht wird, verschwenkt werden kann, was insbesondere bei sehr hochstehendem kindlichen Kopf im Geburtskanal vorteilhaft sein kann.

Schließlich ist es auch denkbar, die Kugelgelenkanordnung so auszugestalten, daß die Kugeln 16 derselben nicht, wie dies in Figur 3 dargestellt ist, im Endbereich 44, sondern in der Wand 15 gehalten werden.

## Patentansprüche

1. Vakuum-Extraktor zur Geburtshilfe mit einem, eine nach einer Seite offene, evakuierbare Behälterkammer aufweisenden Behälter, an dem eine Zugvorrichtung befestigt ist, dadurch gekennzeichnet, daß der Behälter (1) in wenigstens zwei zur einen Seite offene, getrennt voneinander evakuierbare Behälterkammern (1', 1") aufgeteilt ist.

2. Extraktor nach Anspruch 1, dadurch gekennzeichnet, daß der Behälter (1) durch eine Wand (2) in zwei Behälterkammern (1, 1') aufgeteilt ist.

3. Extraktor nach Anspruch 2, dadurch gekennzeichnet, daß der Behälter (1) zur Bildung einer inneren Behälterkammer (1") und einer dazu konzentrisch angeordneten äußeren Behälterkammer (1') durch eine ringförmige Wand (2) unterteilt ist.

4. Extraktor nach Anspruch 3, dadurch gekennzeichnet, daß der Behälter (1) aus einer oberen Wand (1) und einer an diese angeformten Seitenwand (4) besteht und daß die Wand (2) im Inneren des Behälters (1) an die obere Wand (1) angeformt ist.

5. Extraktor nach Anspruch 4, dadurch gekennzeichnet, daß die Wand (2) und die Seitenwand (4) im wesentlichen die Form von hohlen Kreiszylindern aufweisen.

6. Extraktor nach Anspruch 4 oder 5, dadurch gekennzeichnet, daß das untere freie Ende der Seitenwand (4) entsprechend der Form des Kopfes (K) eines Kindes weiter von der oberen Wand (3) entfernt ist als das untere freie Ende der Wand (2).

7. Extraktor nach einem der Ansprüche 4 bis 5, dadurch gekennzeichnet, daß durch die obere Wand (3) ein Saugstutzen (5) zur äußeren Behälterkammer (1') und ein weiterer Saugstutzen (7) zur inneren Behälterkammer (1") verlaufen.

8. Extraktor nach einem der Ansprüche 4 bis 7, dadurch gekennzeichnet, daß die obere Wand (3) in eine erste obere Wand (3') und eine zweite davon beabstandete zweite obere Wand (3") aufgeteilt ist, die teilweise unterhalb der ersten oberen Wand (3') verläuft, daß an die zweite Wand (3") die Wand (2) angeformt ist, daß an die erste Wand (3') die Seitenwand (4) angeformt ist, daß ein erster Saugstutzen (5) an die erste Wand (3') angeformt ist und über den Zwischenraum zwischen der ersten Wand (3') und der zweiten Wand (3") eine Verbindung zur ersten Behälterkammer (1') herstellt, und daß ein zweiter Saugstutzen (7) mit der zweiten Wand (3") verbunden ist, eine Verbindung zur zweiten Behälterkammer (1") herstellt und dicht durch die erste Wand (3') hindurchgeführt ist.

9. Extraktor nach Anspruch 8, dadurch gekennzeichnet, daß der erste Saugstutzen (5) und der zweite Saugstutzen (7) nahe beieinander und symmetrisch zur Längsachse des Behälters (1) angeordnet sind.

10. Extraktor nach Anspruch 8 oder 9, dadurch gekennzeichnet, daß der zweite Saugstutzen (7) in dem Saugstutzen (5) angeordnet und von diesem beabstandet gehalten wird und daß wenigstens die Längsachse des ersten Saugstutzens (5) entlang der Längsachse der Kammer (1) verläuft.

11. Extraktor nach einem der Ansprüche 1 bis 10, dadurch gekennzeichnet, daß der Behälter (1) über eine mittig an ihn angeformte Kette (9) mit einem Haltegriff (10) verbunden ist.

12. Extraktor nach einem der Ansprüche 1 bis 11, dadurch gekennzeichnet, daß der Behälter (1) mit einem eine Zugstange aufweisenden Haltegriff (10') verbunden ist, der vom Behälter (1) derart mechanisch entkoppelt ist, daß er in Bezug auf diesen eine Kreiselbewegung ausführen kann und um seine Längsachse drehbar ist.

13. Extraktor nach Anspruch 12, dadurch gekennzeichnet, daß das dem Behälter (1) zugewandte Ende der Zugstange des Haltegriffes (10') ein erweitertes Ende (14) mit einer kugelförmigen Außenfläche aufweist, daß von der oberen Wand (3') des Behälters eine in Richtung auf den Haltegriff (10') führende, vorzugsweise etwa kugelförmig ausgestaltete Wand (15) vorgesehen ist, die die kugelförmige Außenfläche des erweiterten Endes (14) des Haltegriffes (10') umgibt, daß zwischen dem erweiterten Ende (14) des Haltegriffes (10') und dem dieses umgebenden Endbereich der Wand (15) eine Kuggellageranordnung (16) derart vorgesehen ist, daß die kugelförmigen Außenfläche des erweiterten Endes (14) des Haltegriffes (10') zugewandte Innenfläche des oberen Endbereiches der Wand (15) an der kugelförmigen Außenfläche des Endes (14) abrollen bzw. gleiten kann und daß der obere Endbereich der Wand (15) so bemessen ist, daß das erweiterte Ende (14) des Haltegriffes (10') in ihm gehalten wird.

14. Extraktor nach Anspruch 13, dadurch gekennzeichnet, daß die Kugellageranordnung (16) am erweiterten Ende (14) befestigt ist.

15. Extraktor nach Anspruch 13 oder 14, dadurch gekennzeichnet, daß der Bereich, in dem sich der obere Endbereich der Wand (15) und das erweiterte Ende (14) des Haltegriffes (10') überlappen von einer elastischen Muffe umgeben ist.

16. Extraktor nach einem der Ansprüche 13 bis 15, dadurch gekennzeichnet, daß der Haltegriff (10') eine Längsbohrung aufweist, durch die den ersten Saugstutzen (5) und den zweiten Saugstutzen (7) mit einer Vakuumeinrichtung verbindende Schläuche verlaufen.

17. Extraktor nach einem der Ansprüche 1 bis 16, dadurch gekennzeichnet, daß eine Dornanordnung (50) vorgesehen ist, die bei der Ausbildung des Vakuums eine vorgegebene Strecke in den kindlichen Skalp eingedrückt wird, und daß die Dornanordnung (50) mit einer Meßeinrichtung zur Ermittlung von Herzaktionen verbindbar ist.

18. Extraktor nach Anspruch 17, dadurch gekennzeichnet daß die Dornanordnung (50) im mittleren Bereich der oberen Wand (3', 3") derart angeordnet ist, daß sie in die innere Behälterkammer (1") hineinragt.

19. Extraktor nach Anspruch 16 und Anspruch 17 oder 18, dadurch gekennzeichnet, daß die Dornanordnung (50) über eine elektrische Leitung (51), die durch die Längsbohrung des Haltegriffes (10') verläuft, mit der Meßeinrichtung verbindbar ist.

20. Extraktor nach einem der Ansprüche 17 bis 19, dadurch gekennzeichnet, daß die Dornanordnung (50) aus wenigstens einem Stift besteht.

21. Extraktor nach einem der Ansprüche 4 bis 20, dadurch gekennzeichnet, daß der Abstand zwischen der Wand (2) und der Seitenwand (4) so bemessen und die Form der an die Öffnungsränder der Wand (2) und der Seitenwand (4) angrenzenden Bereiche so beschaffen ist, daß in Abhängigkeit vom Vakuum in der äußeren Behälterkammer (1') in deren Öffnungsbereich Kopfschwarte des Kopfes (K) derart eingeführt wird, daß das Vakuum in der äußeren Behälterkammer (1') durch Herstellen einer Verbindung zur Umgebung sicher abgebaut wird, bevor sich die innere Behälterkammer (1") von der aufgrund des in ihr erzeugten Vakuums in sie eingeführten Kopfschwarte des Kopfes (K) zu lösen beginnt, und daß beim Abbau des Vakuums in der äußeren Behälterkammer (1') ein Warnsignal erzeugbar ist.

22. Extraktor nach einem der Ansprüche 13 bis 21, dadurch gekennzeichnet, daß die Zugstange des Haltegriffes (13) ein halbstarres Rohr ist.

23. Extraktor nach einem der Ansprüche 1 bis 22, dadurch gekennzeichnet, daß an der Zugstange des Haltegriffes (10') in Richtung auf die Zugstange klappbare Zuggriffteile angeordnet sind.

24. Extraktor nach einem der Ansprüche 1 bis 23, dadurch gekennzeichnet, daß der mittlere Bereich der oberen Wand (3) bzw. die mittleren Bereiche der oberen Wände (3', 3") in Richtung auf die Kammeröffnungen abgesenkt sind.

**Claims**

1. Obstetric vacuum extractor comprising a receptacle having an evacuatable receptacle chamber which is open to one side, said receptacle having traction means attached thereto, characterized in that receptacle (1) is divided into at least two receptacle chambers (1', 1") which are open to said one side and adapted to be evacuated separately.

2. Extractor as in claim 1, characterized in that receptacle (1) is divided into two receptacle chambers (1', 1") by a partition (2).

3. Extractor as in claim 2, characterized in that receptacle (1) is divided by an annular partition (2) to form concentric inner and outer receptacle chambers (1", 1').

4. Extractor as in claim 3, characterized in that receptacle (1) consists of a top wall (1) and of a sidewall (4) connected therewith, and in that partition (2) is connected to top wall (1) inside receptacle (1).

5. Extractor as in claim 4, characterized in that partition (2) and sidewall (4) essentially have the form of hollow circular cylinders.

6. Extractor as in claim 4 or 5, characterized in that the bottom free end of partition (4) is farther away from top wall (3) than the bottom free end of partition (2) in accordance with the shape of an infant head (K).

7. Extractor as in any one of claims 4 to 5, characterized in that top wall (3) has extending therethrough a suction tube (5) to outer receptacle chamber (1') and another suction tube (7) to inner receptacle chamber (1").

8. Extractor as in any one of claims 4 to 7, characterized by top wall (3) being divided into a first top wall (3') and a second top wall (3") which is spaced from and partly underlies said first top wall (3'), by partition (2) being attached to second wall (3"), by sidewall (4) being attached to first wall (3'), by a first suction tube (5) being attached to first wall (3') for establishing communication with first receptacle chamber (1') through the space between first and second walls (3', 3"), and by a second suction tube (7) being connected with second wall (3") to establish communication with second receptacle chamber (1") and extending through first wall (3') in a sealed relationship.

9. Extractor as in claim 8, characterized by first and second suction tubes (5) and (7) being disposed close to each other and symmetrically relative to the longitudinal axis of receptacle (1).

10. Extractor as in claim 8 or 9, characterized by second suction tube (7b) being disposed inside suction tube (5) in a spaced relationship thereto and in that the longitudinal axis of at least first suction tube (5) extends along the longitudinal axis of chamber (1).

11. Extractor as in any one of claims 1 to 10, characterized in that receptacle (1) is coupled to a holding handle (10) via a chain (9) centrally attached to said receptacle.

12. Extractor as in any one of claims 1 to 11, characterized by receptacle (1) being connected to a holding handle (10') having a pull rod associated therewith and being mechanically decoupled from receptacle (1) in a manner to allow gyrating movement relative thereto and rotation about its longitudinal axis.

13. Extractor as in claim 12, characterized in that end of the pull rod of handle (10') which is directed towards receptacle (1) having an enlarged end (14) having a spherical exterior surface, by a wall (15) which extends from top wall (3') of the receptacle towards handle (10') and preferably is substantially spherical in shape and encloses said spherical exterior surface of enlarged end (14) of handle (10'), by ball bearing means (16) being provided between enlarged end (14) of handle (10') and the end portion of wall (15) enclosing it in such a manner that the interior surface of the top end portion of wall (15) opposite the exterior surface of enlarged end (14) of handle (10') may roll off or slide on the spherical exterior surface of end (14), and by the top end portion of wall (15) being dimensioned to retain the enlarged end (14) of handle (10') therein.

14. Extractor as in claim 13, characterized by ball bearing means (16) being secured to enlarged end (14).

15. Extractor as in claim 13 or 14, characterized by a flexible sleeve enclosing the area where the top end portion of wall (15) overlies enlarged end (14) of handle (10').

16. Extractor as in any one of claims 13 to 15, characterized by handle (10') having a longitudinal bore therethrough through which extend flexible tubes connecting first and second suction tubes (5) and (7) to vacuum means.

17. Extractor as in any one of claims 1 to 16, characterized by pin means (50) adapted to penetrate a predetermined distance into the infant scalp as a vacuum is being established, said pin means (50) being adapted to be connected with measuring means for detecting cardiac reactions.

18. Extractor as in claim 17, characterized by pin means (50) being disposed in the central area of top wall (3', 3") in a manner to protrude into inner receptacle chamber (1").

19. Extractor as in claim 16 and in claim 17 or 18, characterized by pin means (50) being adapted to be connected to said measuring means by an electric lead (51) extending through the longitudinal bore in handle (10').

20. Extractor as in any one of claims 17 to 19, characterized by pin means (50) comprising at least one pin.

21. Extractor as in any one of claims 4 to 20, characterized by the distance between partition (2) and side wall (4) being dimensioned and the regions adjacent the free edges of partition (2) and side wall (4) being contoured to introduce the scalp of head (K) into the opening region of outer receptacle chamber (1') in dependence on the vacuum therein in such a manner that by establishing a communication to ambient the vacuum in outer receptacle chamber (1') will be safely abated before inner receptacle chamber (1") starts to disengage from the scalp of head (K) which was introduced thereinto by the vacuum generated in it, and by an alarm being generatable while the vacuum in outer receptacle chamber (1') is being abated.

22. Extractor as in any one of claims 13 to 21, characterized by the pull rod of handle (13) being a semi-rigid tube.

23. Extractor as in any one of claims 1 to 22, characterized by the pull rod of handle (10') having associated therewith handle portions adapted to be swung down to lie against the pull rod.

24. Extractor as in any one of claims 1 to 23, characterized by the central area of top wall (3) or the central areas of top walls (3', 3") being depressed towards the chamber openings.

**Revendications**

1. Ventouse obstétricale comprenant un récipient ayant une chambre évacuable et ouverts vers un côté, et un dispositif de tirage attaché audit récipient, caractérisée en ce que ledit récipient (1) est divisé en au moins deux chambres de récipient (1', 1") qui sont ouvertes vers le côté et évacuables séparament.

2. Ventouse obstétricale selon la revendication 1, caractérisée en ce que le récipient (1) est divisé en deux chambres de récipient (1', 1") par un cloison (2).

3. Ventouse obstétricale selon la revendication 2, caractérisée en ce que le récipient (1) est divisé par un cloison annulaire (2) de sorte de former chambres de récipient concentriques intérieure (1") et extérieure (1').

4. Ventouse obstétricale selon la revendication 3, caractérisée en ce que le récipient (1) comprend un paroi supérieur (1) et un paroi latéral (4) formé solidaire avec le paroi (1), et en ce que le cloison (2) est formé solidaire avec le paroi supérieur (1) à l'intérieur de récipient (1).

5. Ventouse obstétricale selon la revendication 4, caractérisée en ce que le cloison (2) et le paroi latéral (4) ont essentiellement la forme de cylindres creux circulaires.

6. Ventouse obstétricale selon la revendication 4 ou 5, caractérisée en ce que la terminaison libre inférieure du paroi latéral (4) se trouve à une distance plus grande du paroi supérieur (3) que la terminaison inférieure du cloison (2) selon la forme de la tête (K) d'un enfant.

7. Ventouse obstétricale selon une des revendications 4 à 5, caractérisée par une tubulure d'aspiration (5) pénetrant le paroi (3) vers la chambre de récipient extérieure (1') et une tubulure d'aspiration (7) pénetrant ledit paroi vers la chambre de récipient intérieure (1").

8. Ventouse obstétricale selon une des revendications 4 à 7, caractérisée en ce que le paroi (3) est divisé en un premier paroi supérieur (3') et un second paroi inférieur (3") espacé du premier paroi et s'étendant sous une partie de celui-ci, que le cloison (2) est formé solidaire avec le second paroi (3"), que le paroi latéral (4) est formé solidaire avec le premier paroi (3'), qu'une première tubulure d'aspiration (5) est formée solidaire avec le premier paroi (3') et établit à travers l'espace entre les parois premier et second (3', 3") une communication à la première chambre de récipient (1'), et qu'une seconde tubulure d'aspiration (7) est formée solidaire avec le second paroi (3"), établit une communication à la seconde chambre de récipient (1") et traverse le premier paroi (3') de façon étanche.

9. Ventouse obstétricale selon la revendication 8, caractérisée en ce que la première tubulure d'aspiration (5) et la seconde tubulure d'aspiration (7) se trouvent l'une à proximité de l'autre et en symmétrie par rapport à l'axe longitudinale du récipient (1).

10. Ventouse obstétricale selon la revendication 8 ou 9, caractérisée en ce que la seconde tubulure d'aspiration (7) est arrangée à l'intérieur de la tubulure d'aspiration (5) et est tenue espacée par rapport à celle-ci, et qu'au moins l'axe longitudinale de la première tubulure d'aspiration (5) s'étend le long de l'axe longitudinale de la chambre (1).

11. Ventouse obstétricale selon une des revendications 1 à 10, caractérisée en ce que le récipient (1) est lié à une poignée (10) à l'aide d'une chaîne (9) attachée au récipient au centre de celui-ci.

12. Ventouse obstétricale selon une des revendications 1 à 11, caractérisée en ce que le récipient (1) est lié à une poignée (10') portant un barreau de tirage qu'elle est découplée du récipient (1) mécaniquement de sorte qu'elle peut exécuter un mouvement giratoire par rapport à celui-ci et tourner autour de son axe longitudinale.

13. Ventouse obstétricale selon la revendication 12, caractérisée en ce que celle des extrémités du barreau de tirage de la poignée (10') qui est dirigée vers le récipient (1) est pourvue d'un élargissement (14) ayant une surface extérieure sphérique, qu'il est pourvu un paroi (15) qui s'étend du paroi supérieur (3') du récipient vers la poignée (10') et a une forme de préférence sphérique, ledit paroi (15) entourant la surface extérieure sphérique de l'extrémité élargie (14) de la poignée (10'), qu'est pourvu entre l'extrémité élargie (14) de la poignée (10') et la partie extrême du paroi (15) entourant celle-ci un roulement à billes (16) de sorte que la surface intérieure de la partie supérieure du paroi (15) en regard de l'élargissement (14) de la poignée (10') peut dérouler ou glisser sur la surface extérieure sphérique de l'extrémité élargie (14), et en ce que la partie extrême supérieure du paroi (15) est dimensionné de sorte qu'il puisse retenir à son intérieur l'extrémité élargie (14) de la poignée (10').

14. Ventouse obstétricale selon la revendication 13, caractérisée en ce que le roulement à billes (16) est arrangé sur l'extrémité élargie (14).

15. Ventouse obstétricale selon la revendication 13 ou 14, caractérisée en ce que la région où la partie supérieure extrême du paroi (15) et l'extrémité élargie (14) de la poignée (10') se recouvrent est entourée d'une gaine élastique.

16. Ventouse obstétricale selon une des revendications 13 à 15, caractérisée en ce que la poignée (10') contient un alésage longitudinal où s'étendent des tubes flexibles liant les tubulures d'aspiration (5) et (7) à un dispositif de vide.

17. Ventouse obstétricale selon une des revendications 1 à 16, caractérisée par un dispositif de tiges (50) destiné à être enfoncé d'une certaine distance dans le scalp de l'enfant pendant que le vide est développé, ledit dispositif de tiges (50) pouvant être lié à moyens pour mesurer des réactions cardiaques.

18. Ventouse obstétricale selon la revendication 17, caractérisée en ce que le dispositif de tiges (50) est arrangé dans la région centrale du paroi supérieur (3', 3") de façon que le dispositif de tiges puisse pénétrer à l'intérieur de la chambre de récipient intérieure (1").

19. Ventouse obstétricale selon les revendications 16 et 17 ou 18, caractérisée en ce que le dispositif de tiges (50) est liable aux moyens de mesure par une conduite électrique (51) menée le long de l'alésage longitudinal de la poignée (10').

20. Ventouse obstétricale selon une des revendications 17 à 19, caractérisée en ce que le dispositif de tiges (50) comprend un tige au moins.

21. Ventouse obstétricale selon une des revendications 4 à 20, caractérisée en ce que la distance entre le cloison (2) et le paroi latéral (4) est telle, et que les contours des régions voisines des ouvertures du cloison (2) et du paroi (4) sont tels, que le scalp de la tête infantine (K) est introduit dans la région d'ouverture de la chambre de récipient extérieure (1') selon le vide établi dans cette chambre de façon que, en établissant une communication à l'environnement, le vide dans la chambre de récipient extérieure (1') est abattu sûrement avant que la chambre de récipient intérieure (1") commence à se détacher du scalp de la tête (K) introduit dans cette chambre par le vide établi dans celle-ci, et en ce que un signal avertisseur peut être produit pendant l'abattement du vide dans la chambre de récipient extérieure (1').

22. Ventouse obstétricale selon une des revendications 13 à 21, caractérisée en ce que le barreau de tirage de la poignée (13) comprend un tuyau sémi-rigide.

23. Ventouse obstétricale selon une des revendications 1 à 22, caractérisée en ce que la poignée (10') comporte des parts basculant vers le barreau de tirage de celle-ci.

24. Ventouse obstétricale selon une des revendications 1 à 23, caractérisée en ce que la région centrale du paroi supérieur (3) ou les régions centrales des parois supérieurs (3', 3") sont incurvées en direction vers les ouvertures des chambres.

FIG. 1

FIG. 2

FIG. 5

FIG. 3

FIG. 4